# EUROPEAN PATENT SPECIFICATION

(11) **EP 2 118 287 B1**
(45) Date of publication and mention of the grant of the patent: **05.11.2014**
(21) Application number: 08720073.9
(22) Date of filing: 07.01.2008
(51) Int. Cl.: C12N 15/82, C12N 5/14

(54) **A CHIMERIC PROMOTER AND A METHOD THEREOF**
CHIMÄRER PROMOTER UND VERFAHREN DAFÜR
PROMOTEUR CHIMERE ET PROCEDE ASSOCIE

(30) Priority: 05.01.2007 IN CH00302007
(43) Date of publication of application: 18.11.2009
(73) Proprietor: Metahelix Life Sciences Private Limited, Bangalore, Karnataka 560 099 (IN)
(72) Inventor: KUMAR, Anil, Bangalore, Karnataka 560 099 (IN); GUPTA, Sonika, Bangalore, Karnataka 560 099 (IN); MUKUNDAN, Sampath, Bangalore, Karnataka 560 099 (IN); DATTAROY, Tomal, Krishna, Bangalore, Karnataka 560 099 (IN); RAMANATHAN, Vairamani, Bangalore, Karnataka 560 099 (IN)
(74) Representative: Ehnis, Tobias
(86) International application number: PCT/IN2008/000004
(87) International publication number: WO 2008/081478

(56) References cited:
- US-A1- 2004 088 760
- US-A1- 2005 283 856
- US-A1- 2006 277 628
- SAGLIOCCO F ET AL: "SEQUENCE OF AN RBCS GENE FROM COTTON" PLANT MOLECULAR BIOLOGY, SPRINGER, DORDRECHT, NL, vol. 17, 1 January 1991 (1991-01-01), XP001055025 ISSN: 0167-4412
- SONG P ET AL: "MOLECULAR BIOLOGY EXPRESSION OF TWO TISSUE-SPECIFIC PROMOTERS IN TRANSGENIC COTTON PLANTS" JOURNAL OF COTTON SCIENCE, COTTON FOUNDATION, MEMPHIS, TN, US, vol. 4, no. 4, 1 January 2000 (2000-01-01) , pages 217-223, XP001055027
- KAY R ET AL: "DUPLICATION OF CAMV 35S PROMOTER SEQUENCES CREATES A STRONG ENHANCER FOR PLANT GENES" SCIENCE, AMERICAN ASSOCIATION FOR THE ADVANCEMENT OF SCIENCE, WASHINGTON, DC; US, vol. 236, no. 4806, 5 June 1987 (1987-06-05), pages 1299-1302, XP000944658 ISSN: 0036-8075

## Description

### FIELD OF THE INTENTION

The invention is in the area of agricultural biotechnology. The present invention provides a novel promoter system that is a fusion of domains of two different promoters. It relates to a chimeric promoter of SEQ ID No. I constructed by fusion of 5' end of GhrbcSP with minimal promoter from 3' end of D35SP. It also relates to a vector carrying said promoter, a method to obtain said promoter, a method of expressing genes using said promoter and a host carrying the promoter. The chimeric promoter of the instant invention is expressed in all tissues except seed endosperm thereby providing for expression of genes coding for proteins essential for crop improvement in a tissue specific manner and yet developing a crop fit for consumption, preferably for human consumption.

### BACKGROUND AND PRIOR ART OF THE INVENTION

This is a gene promoter system that enhances the expression of a transgene by virtue of its property of having the essential elements of different promoters fused together.

The promoters in eukaryotic cells have a 'TATA box', or initiator, close to the transcription start site, typically about 35 bases upstream of it. Different promoters, in isolation, although known to be constitutive, have different levels of expression of the genes they are driving. A reporter gene may express more under the influence of a particular promoter than another.

Fusion of elements of different promoters may result in enhanced expression of the desirable traits in transgenic plants. Some evidences of this have been seen in literature.

Fused plant promoters are known to exist in literature. The examples are:
(a) Fusion of the *Arabidopsis* actin promoter elements at the 5' end with elements of the elongation factor 1 alpha (EF1α) promoter at the 3' end (1),
(b) Fusion of the Figwort mosaic virus promoter elements at the 5' end with elements of EFIα promoter at the 3' end (2),
(c) Fusion of the CaMV 35S promoter elements at the 5' end with elements of the *Arabidopsis* actin 8 promoter at the 3' end (3).

Synthetic promoters with upstream activating regions from the maize Ubi-I gene have been tested (4, 5). Domain swapping, wherein the minimal promoter regions from heterologous promoters from pea, *Arabidopsis* and petunia were fused to the 5' end of CaMV 35S promoter, yielded enhanced expression (6). Chemically synthesized artificial promoters with signature sequences also have shown increased expression (7, 8).

A novel fused promoter (designated as 'super promoter') for high expression in plants uses elements from two bacterial promoters that have been fused (9). The elements are from the octopine synthase promoter and the mannopine synthase promoter from *Agrobacterium tumefaciens.*

The present invention provides a chimeric promoter constructed by fusion of GhrbcSP and D35SP promoters for tissue specific expression of genes which is not known in the prior art. The chimeric promoter of the instant invention is expressed in all tissues except seed endosperm thereby providing for expression of genes coding for proteins essential for crop improvement in a tissue specific manner and yet developing a crop fit for consumption, preferably for human consumption.

### OBJECT OF THE INVENTION

The main object of the present invention is to develop a chimeric promoter for tissue specific expression of genes.

Yet another main object of the present invention is to develop a chimeric promoter constructed by fusion of 5' end of GhrbcSP with minimal promoter from 3' end of D35SP.

Still another object of the present invention is to develop a vector carrying chimeric promoter.

Still another object of the present invention is to develop a vector carrying the expression cassette chimeric promoter-target gene.

Still another main object of the present invention is to develop a method to obtain chimeric promoter.

Still another main object of the present invention is to develop a method of expressing genes using chimeric promoter in plants.

Still another main object of the present invention is to develop a specific chimeric promoter that expresses across tissues except in the seed endosperm thereby enabling the engineering of food crops with traits that are desirable in tissues other than the seed endosperm.

Still another main object of the present invention is to develop a chimeric promoter that is expressed in all tissues except seed endosperm thereby providing for expression of genes coding for proteins essential for crop improvement in a tissue specific manner and yet developing a crop fit for consumption, preferably for human consumption.

Still another main object of the present invention is to develop a prokaryotic or eukaryotic host cell transformed with the chimeric promoter.

### STATEMENT OF THE PRESENT INVENTION

Accordingly, the present invention relates to a chimeric promoter of SEQ ID No. 1 constructed by fusion of 5' end of GhrbcSP with minimal promoter from 3' end of D35SP; a vector carrying chimeric promoter of SEQ ID No. 1, optionally alongwith target gene(s); a method to obtain chimeric promoter of SEQ ID No. I, wherein the method comprising steps of: (a) digesting pMH89 with BamHI to release GhrbcSP fragment from 5' end; (b) digesting pMH70 with Xbal and EcoRV to release larger fragment carrying 35S minimal promoter along with MCS and 35S 3' region; and (c) ligating the GhrbcSP fragment with the larger fragment of XbaI-EcoRV digested pMH70 to obtain the chimeric promoter of SEQ ID No.1; a method of expressing target gene(s) using chimeric promoter of SEQ ID No. 1 in plants, said method comprising steps of: (a) mobilizing a vector carrying expression cassette chimeric promoter-target gene into *Agrobacterium*; and (b) infecting plant embryos with the *Agrobacterium* for expression of the target gene(s) in transformed plants; a prokaryotic or eukaryotic host cell transformed with chimeric promoter of SEQ ID No. 1.

### BRIEF DESCRIPTION OF ACCOMPANYING DRAWINGS

**Fig 1****:** A chimeric (synthetic) promoter that was developed by fusion of 350 bp of GhrbcSP from 5' end with Minimal promoter from 3' end of D35SP.
**Fig 2**: A Map of pMH210, a binary vector carrying the expression cassette Chimeric Promoter-Intron GUS-nos 3'.
**Fig 3****:** An expected 2.86 kb fragment after digestion of DNA from positive colonies of transformed *E. coli.* Lane 1: pMH210/EcoRI-HindIII ; Lane 2: 1 kb ladder (Fermentas) **Fig 4****:** Plants tested positive for GUS expression in different tissues.
**Fig 5**: (**a**) Staining of Nipponbare leaf tissue section containing Intron - GUS driven by D35S promoter **(b)** Staining of Nipponbare leaf tissue section containing Intron - GUS driven by chimeric promoter
**Fig 6****:** Seedling showing intense blue colouration.
**Fig 7****:** Seeds stained for Gus expression wherein only seed coat took up the stain while the endosperm did not.
**Fig 8****:** A map of pMH150, an intermediate cloning vector where there is a multiple cloning site (MCS) flanked by the chimeric promoter at the 5'end and the 35S3' element at the 3' end.

### DETAILED DESCRIPTION OF THE INVENTION

The present invention relates to a chimeric promoter of SEQ ID No. 1 constructed by fusion of 5' end of GhrbcSP with minimal promoter from 3' end of D35SP.

In another embodiment of the present invention, the chimeric promoter contains about 350bp of 5' end of GhrbcSP.

In yet another embodiment of the present invention, the chimeric promoter contains about 130bp minimal promoter from 3' end of D35SP.

In still another embodiment of the present invention, the chimeric promoter is a plant promoter.

The present invention also relates to a vector carrying chimeric promoter of SEQ ID No. 1, optionally alongwith target gene(s).

In still another embodiment of the present invention, the vector carrying the chimeric promoter is pMH 150.

In still another embodiment of the present invention, the vector pMH150 is an intermediate cloning vector where there is a multiple cloning site (MCS) flanked by the Chimeric Promoter at the 5' end and the 35S3' element at the 3' end.

In still another embodiment of the present invention, the vector carrying the chimeric promoter-target gene(s) is a binary vector pMH210.

In still another embodiment of the present invention, the vector pMH210 is a plant vector. The present invention also relates to a method to obtain chimeric promoter of SEQ ID No. I, wherein the method comprising steps of:
a) digesting pMH89 with BamHI to release GhrbcSP fragment from 5' end;
b) digesting pMH70 with Xbal and EcoRV to release larger fragment carrying 35S minimal promoter along with MCS and 35S 3' region; and
c) ligating the GhrbcSP fragment with the larger fragment of XbaI-EcoRV digested pMH70 to obtain the chimeric promoter of SEQ ID No.1.

In still another embodiment of the present invention, the chimeric promoter contains about 350bp of 5' end of GhrbcSP.

In still another embodiment of the present invention, the chimeric promoter contains about 130bp minimal promoter from 3' end of D35SP.

In still another embodiment of the present invention, the chimeric promoter is a plant promoter.

The present invention also relates to a method of expressing target gene(s) using chimeric promoter of SEQ ID No. 1 in plants, said method comprising steps of:
a) mobilizing a vector carrying expression cassette chimeric promoter-target gene into *Agrobacterium;* and
b) infecting plant embryos with the *Agrobacterium* for expression of the target gene(s) in transformed plants.

In still another embodiment of the present invention, the chimeric promoter is a plant promoter.

In still another embodiment of the present invention, the vector is a binary vector pMH210.

In still another embodiment of the present invention, the chimeric promoter is expressed in all tissues except seed endosperm thereby providing for expression of genes coding for proteins essential for crop improvement in a tissue specific manner and yet developing a crop fit for consumption, preferably for human consumption.

The present invention also relates to a prokaryotic or eukaryotic host cell transformed with chimeric promoter of SEQ ID No. 1.

In still another embodiment of the present invention, the individual promoters GhrbcSP and D35SP are constitutive promoters, which are expressed all over the plant including seed endosperm. On the other hand, the chimeric promoter of instant invention is expressed in all tissues except seed endosperm thereby providing for expression of genes coding for proteins essential for crop improvement in a tissue specific manner and yet developing a crop fit for consumption, preferably for human consumption. This aspect of the instant invention establishes inventiveness.

The present invention is in relation to a chimeric promoter constructed by fusion of about 350 bp of 5' end of GhrbcSP with about 130bp minimal promoter from 3' end of D35SP.

The sequence of the chimeric promoter of the invention is given in SEQ ID No.1 as follows:

The chimeric promoter is constructed by the method which involves digesting the plasm ids containing GhrbcSP and D35SP and ligating the fragments generated thereof.

The chimeric promoter so obtained is cloned into a vector alongwith the target gene(s) for the expression of target gene(s). The method involves mobilizing the vector into Agrobacterium and infecting the host for the expression of target gene(s). The chimeric promoter expresses across tissues except in the seed endosperm thereby enabling the engineering of food crops with traits that are desirable in tissues other those in seed endosperm. Thus, it provides for expression of genes coding for proteins essential for crop improvement in a tissue specific manner and yet developing a crop fit for consumption, preferably for human consumption.

The invention is further elaborated with the help of following examples. However, these examples should not be construed to limit the scope of the invention.

### EXAMPLES:

### Example 1

### Construction of the chimeric promoter and the vector:

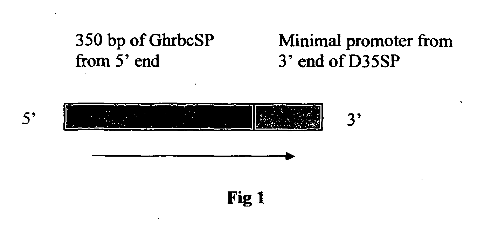

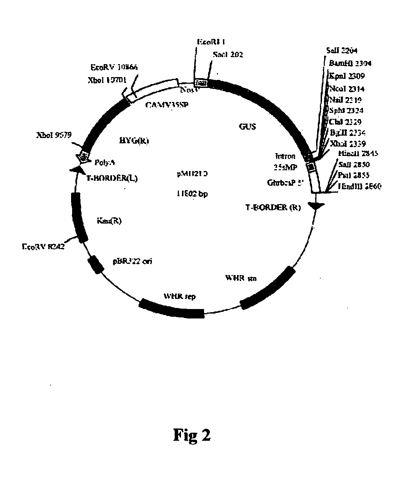

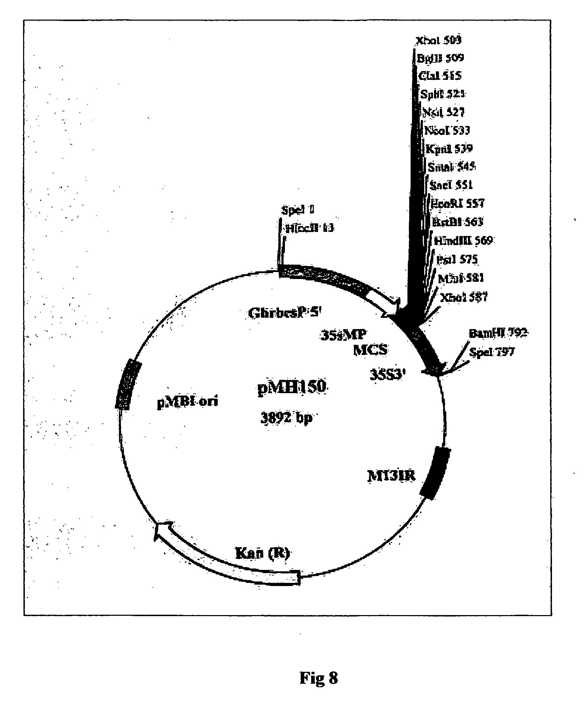

The steps involved in the construction of the chimeric promoter and the vector carrying expression cassette chimeric promoter-target gene, pMH210 is as follows:
- pMH70 is a plasmid carrying an empty expression cassette and is selected on kanamycin. The empty expression cassette has a multiple cloning site (MCS) flanked by a duplicated CaMV 35S promoter (D35SP) upstream of it and a 35S3' polyadenylation signal downstream of it.
- pMH89 is a plasmid clone carrying the Cotton rbcS promoter (GhrbcSP) in pMH65 (a T/A cloning vector).
- pMH93 is a plasmid where a portion encompassing the Intron GUS gene and the nos 3' polyadenylation element (nos3') was cloned as an XbaI-EcoRI fragment into pMH89. Therefore, this is a plasmid that carries an expression cassette wherein GhrbcSP drives Intron GUS gene.
- pMH150: pMH89 was digested with BamHI to release the 350 bp of the GhrbcSP from the 5' end. This fragment was end-filled. pMH70 was digested with Xbal and EcoRV and end-filled. The larger fragment of this end-filled XbaI-EcoRV digested pMH70 carried the 35S minimal promoter along with the MCS and the 35s 3' region. Blunt-end ligation of the 350 bp 5' part of GhrbcSP was carried out with the larger fragment of end-filled XbaI-EcoRV digested pMH70. The resultant plasmid was designated as pMH150 (Fig 8). Assembly of the chimeric promoter (Fig 1) was achieved at this step.
- pMH205: Intron GUS-nos3' fragment was released as an EcoRI fragment from pMH93 and cloned into the EcoRI site of pMH150. The resultant plasmid was designated as pMH205. Assembly of the expression cassette Chimeric Promoter-Intron GUS-nos 3' was achieved at this step.
- pMH210: The expression cassette Chimeric Promoter-Intron GUS-nos 3' was excised as an EcoRI-HindIII fragment from pMH205 and ligated to EcoRI-HindIII digested pMH20, a binary vector. The resultant plasmid was designated as pMH210 (Fig 2), a binary vector carrying the expression cassette Chimeric Promoter-Intron GUS-nos3'.

### Example 2

pMH210 was mobilized into *Agrobacterium,* and the presence of the plasmid was confirmed.

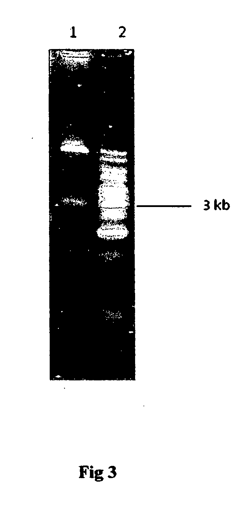

*Agrobacterium tumefaciens* strain EHA105 was electroporated with the plasmid pMH210, and plated on Kanamycin containing medium. Plasmid DNA from the resultant colonies were isolated and re-transformed into *E. coli* DH10B cells which were plated on the same on antibiotic. DNA from positive colonies was subjected to EcoRI and HindIII digestions. An expected 2.86 kb fragment was released as shown in Fig 3.

### Example 3

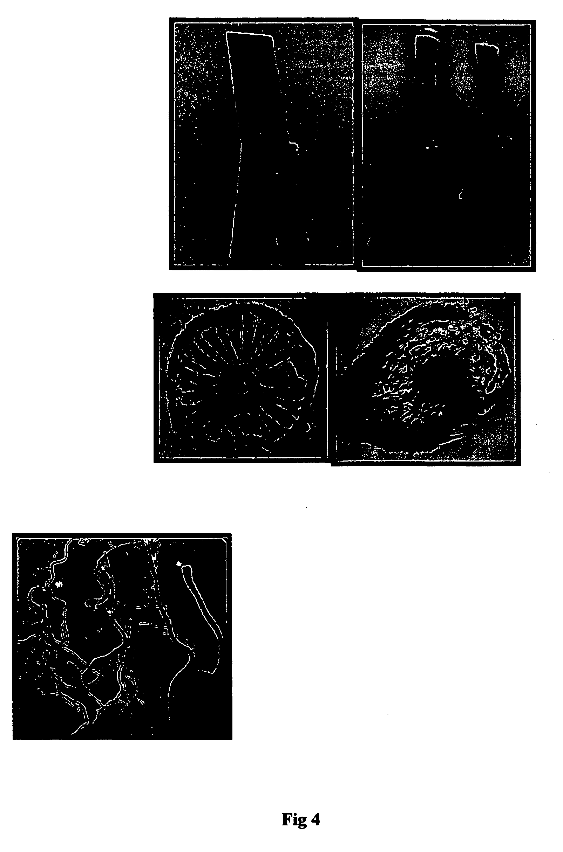

Rice embryos were infected. Seven plants out of four events were selected and these had tested positive for GUS expression in different tissues (Fig 4). The second picture in Fig 4 shows the cross-sections of the roots stained for GUS activity. The left panel has the Intron-GUS driven by D35SP whereas the right panel has the Intron-GUS driven by the chimeric promoter. The intensity of the stain is perceptibly higher in the right panel which shows that enhancement in the expression of genes is due to the chimeric promoter.

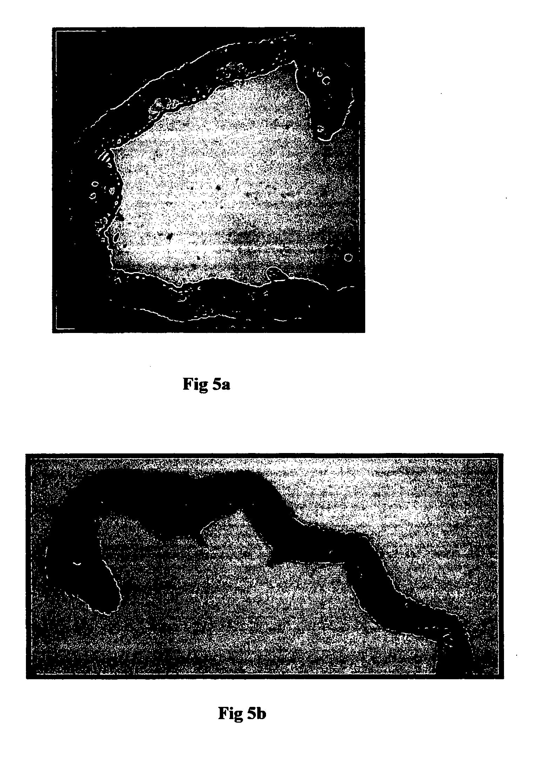

To further elucidate, Fig 5 gives a comparison of the expression between the 2 promoters, viz., the chimeric promoter & the D35SP. Here, Fig 5a has the Intron-GUS driven by D35SP whereas the Fig 5b has the Intron-GUS driven by the chimeric promoter. The intensity of the stain is perceptibly higher in Fig 5b and across the entire section of the leaf tissue, whereas the stain in the tissue where the Intron-GUS gene is driven by the D35SP (Fig 5a) is seen only at a region at the end of the tissue.

### Example 4

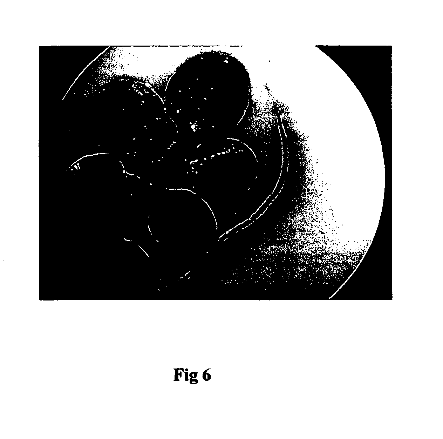

Seeds from tobacco plants were sown and the seedlings showed intense blue coloration (Fig 6) with respect to a control construct.

### Example 5

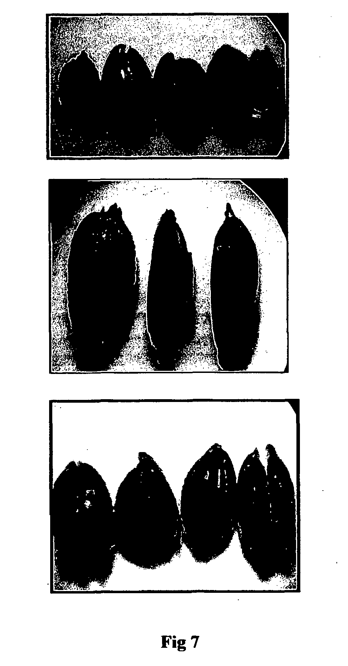

Seeds were stained for GUS expression and it was observed that only the seed coat took up the stain whereas the seed endosperm did not (Fig 7). The second picture in Fig 7 has the D35SP driving the Intron-GUS gene where the entire seed has taken up the stain, whereas the third picture in Fig 7 has the chimeric promoter where only the seed coat has taken up the stain. This qualifies this invention as a unique system for expressing genes in food crops in a way that there is no expression in the edible part, i.e., the seed endosperm.

The aforementioned examples conclusively establish that though, individual promoters GhrbcSP and D35SP are constitutive promoters, which are expressed all over the plant including seed endosperm; but, the chimeric promoter of instant invention is expressed in all tissues except seed endosperm thereby providing for expression of genes coding for proteins essential for crop improvement in a tissue specific manner and yet developing a crop fit for consumption, preferably for human consumption. This aspect of the instant invention establishes inventiveness.

An *e.coli* strain carrying plasmid pMH210 is already deposited in an International Depository Authority [IDA] Microbial Type Culture Collection and gene bank [MTCC], Chandigarh, India.

### ADVANTAGES OF THE INVENTION

- The novel chimeric promoter that uses elements different from the elements used in the prior art. The 5' element of the GhrbcSP as well as the 3' end of the D35SP (minimal promoter) have not been used for any fused promoter constructs earlier.
- This novel chimeric promoter is expressed in all tissues except the seed endosperm (Fig 7). This qualifies this invention as a unique system for expressing genes in food crops in a way that there is no expression in the edible part, i.e., the seed endosperm.

### References:

1. United States Patent 6660911.
2. United States Patent 6949696.
3. United States Patent 6919495.
4. United States Patent 6072050.
5. United States Patent 6555673.
6. Bhullar, S., Chakravarthy, S., Advani, S., Datta, S., Pental, D., Burma, P.K. (2003) Plant Physiology 132, 988-998.
7. United States Patent 6639065.
8. Sawant, S.V., Kiran, K., Mehrotra, R., Chaturvedi, C.P., Ansari, S.A., Singh, P., Lodhi, N., Tuli, R. (2005) J. Exp. Bot., 56, 2345-2353.
9. Ni, M., Cui, D., Einstein, J., Narasimhulu, S., Vergara, C.E., Gelvin, S.B. (1995) Plant J., 7, 661-676.

## Claims

1. A Chimeric promoter of SEQ ID No. 1 constructed by fusion of 5' end of GhrbcSP with minimal promoter from 3' end of D35SP.

2. The chimeric promoter as claimed in claim 1, wherein the chimeric promoter contains about 350bp of 5' end of GhrbcSP.

3. The chimeric promoter as claimed in claim 1, wherein the chimeric promoter is a plant promoter and contains about 130bp minimal promoter from 3' end of D35SP.

4. A vector carrying chimeric promoter of SEQ ID No. 1, optionally along with target gene(s).

5. The vector as claimed in claim 4, wherein the vector carrying the chimeric promoter is pMH150, wherein pMH150 is an intermediate cloning vector having a multiple cloning site (MCS) flanked by the chimeric promoter at the 5' end and a 35S3' element at the 3' end, wherein pMH150 is obtained by digesting pMH89 with BamHI to release 350 bp of the GhrbcSP from the 5' end, end-filling the resultant fragment and blunt-end ligating this fragment with the larger fragment of end-filled XbaI-EcoRV digested pMH70, wherein pMH89 is a plasmid clone carrying the Cotton rbcS promoter (GhrbcSP) in a T/A cloning vector and pMH70 is a plasmid carrying an empty expression cassette and is selected on kanamycin, wherein the empty expression cassette has a multiple cloning site (MCS) flanked by a duplicated CaMV 35S promoter (D35SP) upstream of it and a 35S3' polyadenylation signal downstream of it.

6. The vector as claimed in claim 4, wherein the vector carrying the chimeric promoter-target gene(s) is a binary vector pMH210, wherein pMH210 carries the expression cassette Chimeric Promoter-Intron GUS-nos 3' polyadenylation element, wherein pMH210 is obtained by excising an expression cassette Chimeric Promoter-Intron GUS-nos 3' polyadenylation element as an EcoRI-HindIII fragment from pMH205 and ligating the fragment to a EcoRI-HindIII digested binary vector, wherein pMH205 is obtained by releasing Intron GUS-nos 3' polyadenylation element fragment as an EcoRI fragment from pMH93 and cloning it into the EcoRI site of pMH150 as defined in claim 5, wherein pMH93 is a plasmid where a portion encompassing the Intron GUS gene and the nos 3' polyadenylation element (nos 3') was cloned as an XbaI-EcoRI fragment into pMH89, wherein pMH89 is a plasmid clone carrying the Cotton rbcS promoter (GhrbcSP) in a T/A cloning vector.

7. The vector as claimed in claim 6, wherein the vector pMH210 is a plant vector.

8. A method to obtain Chimeric promoter of SEQ ID No. 1, wherein the method comprising steps of:
a) digesting pMH89 with BamHI to release GhrbcSP fragment from 5' end, wherein pMH89 is a plasmid clone carrying the Cotton rbcS promoter (GhrbcSP) in a T/A cloning vector;
b) digesting pMH70 with Xbal and EcoRV to release larger fragment carrying 35S minimal promoter along with MCS and 35S 3' region, wherein pMH70 is a plasmid carrying an empty expression cassette and is selected on kanamycin, wherein the empty expression cassette has a multiple cloning site (MCS) flanked by a duplicated CaMV 35S promoter (D35SP) upstream of it and a 35S3' polyadenylation signal downstream of it; and
c) ligating the GhrbcSP fragment with the larger fragment of XbaI-EcoRV digested pMH70 to obtain the chimeric promoter of SEQ ID No.1.

9. The method as claimed in claim 8, wherein the chimeric promoter contains about 350 bp of 5' end of GhrbcSP.

10. The method as claimed in claim 8, wherein the chimeric promoter is a plant promoter and contains about 130bp minimal promoter from 3' end of D35SP.

11. A method of expressing target gene(s) using chimeric promoter of SEQ ID No. 1 in plants, said method comprising steps of:
a) mobilizing a vector carrying expression cassette chimeric promoter-target gene into *Agrobacterium;* and
b) infecting plant embryos with the *Agrobacterium* for expression of the target gene(s) in transformed plants.

12. The method as claimed in claim 11, wherein the vector is a binary vector pMH210, wherein pMH210 carries the expression cassette Chimeric Promoter-Intron GUS-nos 3' polyadenylation element, wherein pMH210 is obtained by excising an expression cassette Chimeric Promoter-Intron GUS-nos 3' polyadenylation element as an EcoRI-HindIII fragment from pMH205 and ligating the fragment to a EcoRI-HindIII digested binary vector, wherein pMH205 is obtained by releasing Intron GUS-nos 3' polyadenylation element fragment as an EcoRI fragment from pMH93 and cloning it into the EcoRI site of pMH150 as defined in claim 5, wherein pMH93 is a plasmid where a portion encompassing the Intron GUS gene and the nos 3' polyadenylation element (nos 3') was cloned as an XbaI-EcoRI fragment into pMH89, wherein pMH89 is a plasmid clone carrying the Cotton rbcS promoter (GhrbcSP) in a T/A cloning vector.

13. The method as claimed in claim 11, wherein the chimeric promoter is a plant promoter and is expressed in all tissues except seed endosperm thereby providing for expression of genes coding for proteins essential for crop improvement in a tissue specific manner and yet developing a crop fit for consumption, preferably for human consumption.

14. A prokaryotic or eukaryotic host cell transformed with chimeric promoter of SEQ ID No. 1.

## Patentansprüche

1. Chimärer Promotor von SEQ ID No. 1, welcher durch Fusion des 5'-Endes von GhrbcSP mit dem Minimal-Promotor vom 3'-Ende von D35SP gebaut wurde.

2. Chimärer Promotor nach Anspruch 1, wobei der chimäre Promotor etwa 350 bp des 5'-Endes von GhrbcSP enthält.

3. Chimärer Promotor nach Anspruch 1, wobei der chimäre Promotor ein Pflanzenpromotor ist und etwa 130 bp des Minimal-Promotors vom 3'-Ende von D35SP enthält.

4. Vektor, welcher den chimären Promotor von SEQ ID No. 1, optional zusammen mit Zielgenen/einem Zielgen, trägt.

5. Vektor nach Anspruch 4, wobei der den chimären Promotor tragende Vektor pMH150 ist, wobei pMH150 ein intermediärer Klonierungsvektor ist, welcher eine multiple Klonierungsstelle (MCS) aufweist, welche von dem chimären Promotor am 5'-Ende und einem 35S3'-Element am 3'-Ende flankiert ist, wobei pMH150 erhalten wird durch Verdauen von pMH89 mit BamHI, um 350 bp von dem GhrbcSP vom 5'-Ende freizusetzen, Auffüllen der Enden des erhaltenen Fragments und Ligieren dieses Fragments an den stumpfen Enden mit dem größeren Fragment von einem an den Enden aufgefüllten XbaI-EcoRV-verdauten pMH70, wobei pMH89 ein Plasmidklon ist, welcher den Baumwoll-rbcS-Promotor (GhrbcSP) in einem T/A-Klonierungsvektor trägt und pMH70 ein Plasmid ist, welches eine leere Expressionskassette trägt und auf Kanamycin selektiert wird, wobei die leere Expressionskassette eine multiple Klonierungsstelle (MCS) aufweist, welche von einem duplizierten CaMV 35S-Promotor (D35SP) stromaufwärts davon und einem 35S3'-Polyadenylierungssignal stromabwärts davon flankiert ist.

6. Vektor nach Anspruch 4, wobei der Vektor, welcher die chimären Promotor-Zielgene/das chimäre Promotor-Zielgen trägt, ein binärer pMH210-Vektor ist, wobei pMH210 das Expressionskassette-chimärer Promotor-Intron GUS-nos 3'-Polyadenylierungselement trägt, wobei pMH210 erhalten wird durch Ausschneiden eines Expressionskassettechimärer Promotor-Intron GUS-nos 3'-Polyadenylierungselements als ein EcoRI-HindIII-Fragment aus pMH205 und Ligieren des Fragments an einen EcoRI-HindIII verdauten binären Vektor, wobei pMH205 erhalten wird durch Freisetzen des Intron GUS-nos 3'-Polyadenylierungselement-Fragments als ein EcoRI-Fragment von pMH93 und dessen Klonieren in die EcoRI-Stelle von pMH150, wie in Anspruch 5 definiert, wobei pMH93 ein Plasmid ist, wo ein das Intron GUS-Gen und das nos 3'-Polyadenylierungselement (nos 3') umfassender Anteil als ein XbaI-EcoRI-Fragment in pMH89 kloniert wurde, wobei pMH89 ein den Baumwoll-rbcS-Promotor (GhrbcSP) in einem T/A-Klonierungsvektor tragender Plasmidklon ist.

7. Vektor nach Anspruch 6, wobei der Vektor pMH210 ein Pflanzenvektor ist.

8. Verfahren zum Erhalten des chimären Promotors von SEQ ID No. 1, wobei das Verfahren die folgenden Schritte umfasst:
a) Verdauen von pMH89 mit BamHI um das GhrbcSP-Fragment vom 5'-Ende freizusetzen, wobei pMH89 ein Plasmidklon ist, welcher den Baumwoll-rbcS-Promotor (GhrbcSP) in einem T/A-Klonierungsvektor trägt;
b) Verdauen von pMH70 mit XbaI und EcoRV um ein größeres Fragment freizusetzen, welches den 35S-Minimal-Promotor zusammen mit der MCS und der 35S 3'-Region trägt, wobei pMH70 ein Plasmid ist, welches eine leere Expressionskassette trägt und auf Kanamycin selektiert wird, wobei die leere Expressionskassette eine multiple Klonierungsstelle (MCS) aufweist, welche von einem duplizierten CaMV 35S-Promotor (D35SP) stromaufwärts davon und einem 35S3'-Polyadenylierungssignal stromabwärts davon flankiert wird; und
c) Ligieren des GhrbcSP-Fragments mit dem größeren Fragment von XbaI-EcoRV verdautem pMH70, um den chimären Promotor von SEQ ID No. 1 zu erhalten.

9. Verfahren nach Anspruch 8, wobei der chimäre Promotor etwa 350 bp vom 5'-Ende von GhrbcSP enthält.

10. Verfahren nach Anspruch 8, wobei der chimäre Promotor ein Pflanzenpromotor ist und etwa 130 bp Minimal-Promotor vom 3'-Ende von D35SP enthält.

11. Verfahren des Exprimierens eines Zielgens/von Zielgenen unter Verwendung des chimären Promotors von SEQ ID No. 1 in Pflanzen, wobei das Verfahren Schritte umfasst des
a) Mobilisierens eines Vektors, welcher Expressionskassette-chimärer Promotor-Zielgen trägt, in *Agrobacterium;* und
b) Infizierens von Pflanzenembryonen mit dem *Agrobacterium* zur Expression des Zielgens/der Zielgene in transformierten Pflanzen.

12. Verfahren nach Anspruch 11, wobei der Vektor ein binärer pMH210-Vektor ist, wobei pMH210 das Expressionskassettechimärer Promotor-Intron GUS-nos 3'-Polyadenylierungselement trägt, wobei pMH210 erhalten wird durch Ausschneiden eines Expressionskassette-chimärer Promotor-Intron GUS-nos 3'-Polyadenylierungselements als ein EcoRI-HindIII-Fragment aus pMH205 und Ligieren des Fragments an einen EcoRI-HindIII verdauten binären Vektor, wobei pMH205 erhalten wird durch Freisetzen des Intron GUS-nos 3'-Polyadenylierungselement-Fragments als ein EcoRI-Fragment von pMH93 und dessen Klonieren in die EcoRI-Stelle von pMH150, wie in Anspruch 5 definiert, wobei pMH93 ein Plasmid ist, wo ein das Intron GUS-Gen und das nos 3'-Polyadenylierungselement (nos 3') umfassender Anteil als ein XbaI-EcoRI-Fragment in pMH89 kloniert wurde, wobei pMH89 ein den Baumwoll-rbcS-Promotor (GhrbcSP) in einem T/A-Klonierungsvektor tragender Plasmidklon ist.

13. Verfahren nach Anspruch 11, wobei der chimäre Promotor ein Pflanzenpromotor ist und in allen Geweben außer Samen-Endosperm exprimiert wird, wodurch die Expression von Genen, die für Proteine kodieren, welche für eine Ernteverbesserung in einer gewebespezifischen Art essentiell sind, und dennoch die Entstehung einer zum Verzehr, insbesondere zum menschlichen Verzehr, geeigneten Ernte gewährleistet wird.

14. Prokaryotische oder eukaryotische Wirtszelle, welche mit dem chimären Promotor von SEQ ID No. 1 transformiert ist.

## Revendications

1. Promoteur chimère de SEQ ID N° 1 construit par la fusion de l'extrémité 5' de GhrbcSP avec le promoteur minimal depuis l'extrémité 3' de D35SP.

2. Promoteur chimère selon la revendication 1, dans lequel le promoteur chimère contient environ 350 bp de l'extrémité 5' de GhrbcSP.

3. Promoteur chimère selon la revendication 1, dans lequel le promoteur chimère est un promoteur végétal et contient environ 130 bp de promoteur minimal depuis l'extrémité 3' de D35SP.

4. Vecteur portant le promoteur chimère de SEQ ID N° 1, en option avec un/des gène(s) cible(s).

5. Vecteur selon la revendication 4, dans lequel le vecteur portant le promoteur chimère est pMH150, dans lequel pMH150 est un vecteur de clonage intermédiaire ayant un site de clonage multiple (MCS) flanqué par le promoteur chimère à l'extrémité 5' et un élément 35S3' à l'extrémité 3', dans lequel pMH150 est obtenu en digérant pMH89 avec BamHI pour libérer 350 bp du GhrbcSP de l'extrémité 5', remplissant l'extrémité du fragment résultant et ligaturant l'extrémité franche de ce fragment avec le fragment plus grand de pMH70 digéré avec XbaI-EcoRV à extrémité remplie, dans lequel pMH89 est un clone de plasmide portant le promoteur rbcS de coton (GhrbcSP) dans un vecteur de clonage T/A et pMH70 est un plasmide portant une cassette d'expression vide et est sélectionné sur de la kanamycine, dans lequel la cassette d'expression vide a un site de clonage multiple (MCS) flanqué par un promoteur CaMV 35S dupliqué (D35SP) en amont de celui-ci et un signal de polyadénylation 35S3' en aval de celui-ci.

6. Vecteur selon la revendication 4, dans lequel le vecteur portant le promoteur chimère-le(s) gène(s) cible(s) est un vecteur binaire pMH210, dans lequel pMH210 porte la cassette d'expression promoteur chimère-GUS à intron-élément de polyadénylation en 3' nos, dans lequel pMH210 est obtenu en excisant une cassette d'expression promoteur chimère-GUS à intron-élément de polyadénylation en 3' nos comme un fragment EcoRI-HindIII de pMH205 et ligaturant le fragment à un vecteur binaire digéré avec EcoRI-HindIII, dans lequel pMH205 est obtenu en libérant le fragment GUS à intron-élément de polyadénylation en 3' nos comme un fragment EcoRI de pMH93 et le clonant dans le site EcoRI de pMH150 comme défini à la revendication 5, dans lequel pMH93 est un plasmide où une portion englobant le gène GUS à intron et l'élément de polyadénylation en 3' nos (nos 3') a été clonée comme un fragment XbaI-EcoRI dans pMH89, dans lequel pMH89 est un clone de plasmide portant le promoteur rbcS de coton (GhrbcSP) dans un vecteur de clonage T/A.

7. Vecteur selon la revendication 6, dans lequel le vecteur pMH210 est un vecteur végétal.

8. Procédé d'obtention du promoteur chimère de SEQ ID N° 1, dans lequel le procédé comprenant les étapes consistant à :
a) digérer pMH89 avec BamHI pour libérer le fragment GhrbcSP de l'extrémité 5', dans lequel pMH89 est un clone de plasmide portant le promoteur rbcS de coton (GhrbcSP) dans un vecteur de clonage T/A ;
b) digérer pMH70 avec XbaI et EcoRV pour libérer un fragment plus grand portant le promoteur minimal 35S avec MCS et la région en 3' 35S, dans lequel pMH70 est un plasmide portant une cassette d'expression vide et est sélectionné sur de la kanamycine, dans lequel la cassette d'expression vide a un site de clonage multiple (MCS) flanqué par un promoteur CaMV 35S dupliqué (D35SP) en amont de celui-ci et un signal de polyadénylation 35S3' en aval de celui-ci ; et
c) ligaturer le fragment GhrbcSP avec le fragment plus grand de pMH70 digéré avec XbaI-EcoRV pour obtenir le promoteur chimère de SEQ ID N° 1.

9. Procédé selon la revendication 8, dans lequel le promoteur chimère contient environ 350 bp de l'extrémité 5' de GhrbcSP.

10. Procédé selon la revendication 8, dans lequel le promoteur chimère est un promoteur végétal et contient environ 130 bp de promoteur minimal depuis l'extrémité 3' de D35SP.

11. Procédé d'expression d'un/de gène(s) cible(s) utilisant le promoteur chimère de SEQ ID N° 1 dans des plantes, ledit procédé comprenant les étapes consistant à :
a) mobiliser un vecteur portant une cassette d'expression promoteur chimère-gène cible dans Agrobacterium ; et
b) infecter des embryons végétaux avec l'Agrobacterium pour l'expression du/des gène(s) cible(s) dans des plantes transformées.

12. Procédé selon la revendication 11, dans lequel le vecteur est un vecteur binaire pMH210, dans lequel pMH210 porte la cassette d'expression promoteur chimère-GUS à intron-élément de polyadénylation en 3' nos, dans lequel pMH210 est obtenu en excisant une cassette d'expression promoteur chimère-GUS à intron-élément de polyadénylation en 3' nos comme un fragment EcoRI-HindIII de pMH205 et ligaturant le fragment à un vecteur binaire digéré avec EcoRI-HindIII, dans lequel pMH205 est obtenu en libérant le fragment GUS à intron-élément de polyadénylation en 3' nos comme un fragment EcoRI de pMH93 et le clonant dans le site EcoRI de pMH150 comme défini à la revendication 5, dans lequel pMH93 est un plasmide où une portion englobant le gène GUS à intron et l'élément de polyadénylation en 3' nos (nos 3') a été clonée comme un fragment XbaI-EcoRI dans pMH89, dans lequel pMH89 est un clone de plasmide portant le promoteur rbcS de coton (GhrbcSP) dans un vecteur de clonage T/A.

13. Procédé selon la revendication 11, dans lequel le promoteur chimère est un promoteur végétal et est exprimé dans tous les tissus sauf l'endosperme de graine, fournissant de ce fait l'expression de gènes codant pour des protéines essentielles à l'amélioration de la culture de manière spécifique au tissu et développant toutefois une culture propre à la consommation, de préférence à la consommation humaine.

14. Cellule hôte procaryote ou eucaryote transformée avec le promoteur chimère de SEQ ID N° 1.


### SEQUENCE LISTING

<110> METAHELIX LIFE SCIENCES PRIVATE LIMITED
<120> A CHIMERIC PROMOTER AND A PROCESS THEREOF
<130> PCT0791
<140> PCT/ IN 2008/ 000004
   <141> 2008-01-07
<150> 00030/CHE/2007
   <151> 2007-01-07
<160> 1
<170> PatentIn version 3.5
<210> 1
   <211> 486
   <212> DNA
   <213> GhrbcSP and D35SP
<400> 1
   tgctcatgtt aacaattaat tcctataatc gacatcaaaa ttatatgaaa gaattaacac
   60
   ttggttaccg agttaccata tttgaagata acgcgaaagg taaaaacaca aaaggcaagc
   120
   atgaccaagc aaacaaggta tggacatcga tttttttgaa tcgggaatga ccaaatggga
   180
   ccgtgaagag gtgacaaagg agaaatcagg cattcacggt ttccattgga tgaaatgaga
   240
   taagatcact gtgcttcttc cacgtggcag gttgccaaag ataaggcttt accattcaag
   300
   aaaagtttcc accctctttg tggtcattat ggttgtaatg tcatctgatt taggatcatc
   360
   tccactgacg taagggatga cgcacaatcc cactatcctt cgcaagaccc ttcctctata
   420
   taaggaagtt catttcattt ggagaggaca cgctgaaatc accagtctct ctctacaaat
   480
   ctatct
   486